# EUROPEAN PATENT SPECIFICATION

(11) **EP 4 420 640 B1**
(45) Date of publication and mention of the grant of the patent: **04.03.2026**
(21) Application number: 24181915.0
(22) Date of filing: 25.04.2023
(51) Int. Cl.: A61F 5/443, A61F 5/448

(54) **DYNAMIC CONVEX OSTOMY BARRIER**
DYNAMISCHE KONVEXE OSTOMIEBARRIERE
BARRIÈRE D'OSTOMIE CONVEXE DYNAMIQUE

(30) Priority: 22.06.2022 US 202263354441 P
(43) Date of publication of application: 28.08.2024
(62) Divisional of application: 23724632.7
(73) Proprietor: Hollister Incorporated, Libertyville, IL 60048-3781 (US)
(72) Inventor: CZAPLEWSKI, Gregory J., Libertyville, IL 60048 (US); TETZLAFF, Patrick C., Libertyville, IL 60048 (US); LEADINGHAM, Brian T., Libertyville, IL 60048 (US)
(74) Representative: FRKelly

(56) References cited:
- JP-B2- 5 259 960
- US-A1- 2019 231 580
- US-A1- 2020 253 777
- US-A1- 2022 168 131

## Description

### BACKGROUND

This disclosure is related to a convex ostomy barrier. More particularly, the present disclosure pertains to an ostomy barrier appliance having a localized adjustable convexity feature.

Ostomy pouches for collecting bodily waste are used by individuals who have had surgery such as a colostomy, ileostomy, or urostomy. An ostomy pouch may be attached to a user via an ostomy barrier, which is configured to seal against peristomal skin surfaces and protect the peristomal surfaces from exposure to stomal effluent. However, the topography of stomas and peristomal surfaces surrounding stomas varies among patients, and sealing an ostomy appliance against such different peristomal surfaces and stomas remain as an area for further improvements. For example, a stoma may protrude more or less, or may even be flush or recessed.

A person with an ostomy having a stoma that is flush or recessed may find that applying external support or pressure from a barrier in the peristomal region aids in directing the discharge of effluent from the stoma directly into the ostomy pouch. Accordingly, the effectiveness of an adhesive seal between the ostomy barrier and the peristomal skin surface (i.e., a seal formed by the adhesive layer) may be prolonged. Thus, convex inserts and convex ostomy barriers, such as ADAPT^{®} convex barrier rings available through the Applicant of the present application, have been developed to apply pressure around such peristomal regions.

However, the convexity of a conventional convex ostomy barrier or insert is fixed and may not work efficiently for all ostomates. Thus, convex ostomy barriers and convex inserts of various convexity depths have been made available in the market. Further, US 2019/0231580, which is assigned to the Applicant of the present application, discloses ostomy barrier appliances including a convexity adjusting device, which is configured to allow a user to provide a convexity to a generally flat skin barrier.

Accordingly, it is desirable to provide an improved ostomy barrier appliance that allows local adjustment of various characteristics of the barrier convexity according to the topography of user's stoma and peristomal surface.

### BRIEF SUMMARY

The present disclosure provides a convex ostomy barrier as detailed in claim 1. Advantageous features are provided in dependent claims.

A convexity adjusting device for an ostomy skin barrier configured to adjust a convexity of a skin barrier is provided according to various embodiments.

In one aspect, a convex ostomy barrier assembly for attaching an ostomy appliance to a peristomal skin surrounding a stoma including a skin barrier, an inlet opening defined in the skin barrier for receiving the stoma, and a convexity adjusting device. The convexity adjusting device may include a convex insert and includes an inflatable bladder, wherein the convex insert may be configured to provide a convexity to the skin barrier, and the inflatable bladder may be configured to adjust at least one characteristic of the convexity.

The inflatable bladder is compartmentalized into a plurality of bladders, wherein the convexity adjusting device is configured such that each of the plurality of bladders can be inflated or deflate separately. The convex insert may include a base and an upper layer. The upper layer may extend from the base to an inner periphery, and the inflatable bladder may be arranged adjacent a distal surface of the upper layer between the base and the upper layer. In such an embodiment, the inflatable bladder may be configured to provide a localized adjustment of a compressibility and/or a depth of the convexity by inflating or deflating one or more of the plurality of bladders.

In an embodiment, the ostomy barrier assembly may be provided with each of the plurality of bladders inflated to provide a generally uniform first compressibility around the convexity. In such an embodiment, one or more of the plurality of bladders may be deflated to adjust the first compressibility to a second compressibility at one or more of the corresponding locations to provide a localized and customized adjustment of the compressibility of the convexity, wherein the first compressibility is greater than the second compressibility. In another embodiment, the ostomy barrier assembly may be provided with each of the plurality of bladders deflated to provide a generally uniform third compressibility around the convexity. In such an embodiment, one or more of the plurality of bladders may be inflated to adjust the third compressibility to a fourth compressibility at one or more of the corresponding locations to provide a localized and customized adjustment of the compressibility of the convexity, wherein the third compressibility is less than the fourth compressibility.

In an embodiment, the inflatable bladder may be arranged between the convex insert and the skin barrier, wherein each of the plurality of bladders may be configured to be inflated or deflated separately to provide a localized and customized adjustment of a slope of the convexity. In such an embodiment, the inflatable bladder may be arranged in a concave portion of the convex insert. One or more of the plurality of bladders may be inflated to decrease the slope of the convexity at one or more the corresponding locations or deflated to increase the slope of the convexity at one or more the corresponding locations.

In an embodiment, the convex ostomy barrier assembly may be provided with each of the plurality of bladders filled with air to provide a generally uniform slope around the convexity, wherein one or more of the plurality of bladders may be compressed or popped to increase the slope of the convexity at one or more the corresponding locations.

In an embodiment, the inflatable bladder may be arranged between the convex insert and the skin barrier in a dome area and configured to adjust a depth of the convexity. In such an embodiment, the convex barrier assembly may be provided with the inflatable bladder substantially deflated to provide a first depth, wherein the inflatable bladder may be configured to be inflated by a user to increase the depth to a second depth, wherein the second depth is greater than the first depth. Alternatively, the convex barrier assembly may be provided with the inflatable bladder inflated to provide a third depth, wherein the inflatable bladder may be configured to be deflated by a user to decrease the depth to a fourth depth, wherein the fourth depth is less than the third depth.

In an embodiment, the inflatable bladder may be arranged between the convex insert and the skin barrier in a concave portion of the convex insert and configured to adjust a slope of the convexity. In such an embodiment, the convex barrier assembly may be provided with the inflatable bladder substantially deflated to provide a first slope, wherein the inflatable bladder may be configured to be inflated by a user to decrease the slope to a second slope, wherein the second slope is less than the first slope. Alternatively, the convex barrier assembly may be provided with the inflatable bladder inflated to provide a third slope, wherein the inflatable bladder may be configured to be deflated by a user to increase the slope to a fourth slope, wherein the fourth slope is greater than the third slope.

In an embodiment, the inflatable bladder may be arranged adjacent a distal surface of the convex insert and configured to adjust a compressibility of the convexity. The convex ostomy barrier assembly may be configured such that the compressibility of the convexity can be adjusted by inflating or deflating the inflatable bladder while a depth and a slope of the convexity defined by the convex insert remain unchanged. For example, the inflatable bladder may be configured to be inflated to increase the compressibility of the convexity and deflated to decrease the compressibility of the convexity. In such an embodiment, the inflatable bladder may be arranged proximate a base area of the convex insert.

In any of the foregoing embodiments, the convex ostomy barrier assembly may include a fluid inlet and a fluid path connecting the fluid inlet to the inflatable bladder, wherein the fluid inlet and the fluid path may provide a path for a fluid to enter or exit the inflatable bladder. The inflatable bladder may be configured to be inflated by inserting a fluid into the inflatable bladder and deflated by releasing the fluid from the inflatable bladder. The fluid may be air.

The foregoing general description and the following detailed description are examples only and are not restrictive of the present disclosure.

### BRIEF DESCRIPTION OF THE DRAWINGS

The benefits and advantages of the present embodiments will become more readily apparent to those of ordinary skill in the relevant art after reviewing the following detailed description and accompanying drawings, wherein:
FIG. 1 is a perspective distal side view of an ostomy barrier appliance including a convexity adjusting device comprising a plurality of bladders for localized and customized adjustment of a compressibility and/or depth of a convexity according to an embodiment with a portion removed to illustrate its layered structure.
FIG. 2 is a perspective distal side view of an ostomy barrier appliance including a convexity adjusting device comprising an inflatable bladder for customized adjustment of a convexity depth according to an embodiment with a portion removed to illustrate its layered structure.
FIG. 3A is a schematic cross-section view of the convexity adjusting device of FIG. 2 with a deflated bladder.
FIG. 3B is a schematic cross-section view of the convexity adjusting device of FIG. 2 with an inflated bladder.
FIG. 4A is a schematic cross-section view of a convexity adjusting device comprising an inflatable bladder for customized adjustment of a convexity slope according to another embodiment, wherein the inflatable bladder is deflated.
FIG. 4B is a schematic cross-section view of the convexity adjusting device of FIG. 4A with the inflatable bladder inflated.
FIG. 5A is a schematic cross-section view of a convexity adjusting device comprising an inflatable bladder for customized adjustment of a compressibility according to yet another embodiment with the inflatable bladder deflated.
FIG. 5B is a schematic cross-section view of the convexity adjusting device of FIG. 5A with the inflatable bladder inflated.
FIG. 6 is a perspective distal side view of an ostomy barrier appliance including a convexity adjusting device comprising a plurality of bladders for localized and customized adjustment of a convexity slope according to an embodiment with a portion removed to illustrate its layered structure.
FIG. 7 is an illustration of a depth of a convex skin barrier.
FIG. 8 is an illustration of compressibility of a convex skin barrier.
FIG. 9 is an illustration of flexibility of a convex skin barrier.
FIGS. 10A and 10B are illustrations of tension locations of a convex skin barrier.
FIG. 11 is an illustration of a slope of a convex skin barrier.

### DETAILED DESCRIPTION

While the present disclosure is susceptible of embodiment in various forms, there is shown in the drawings and will hereinafter be described presently preferred embodiments with the understanding that the present disclosure is to be considered an exemplification and is not intended to limit the disclosure to the specific embodiments illustrated. The words "a" or "an" are to be taken to include both the singular and the plural. Conversely, any reference to plural items shall, where appropriate, include the singular.

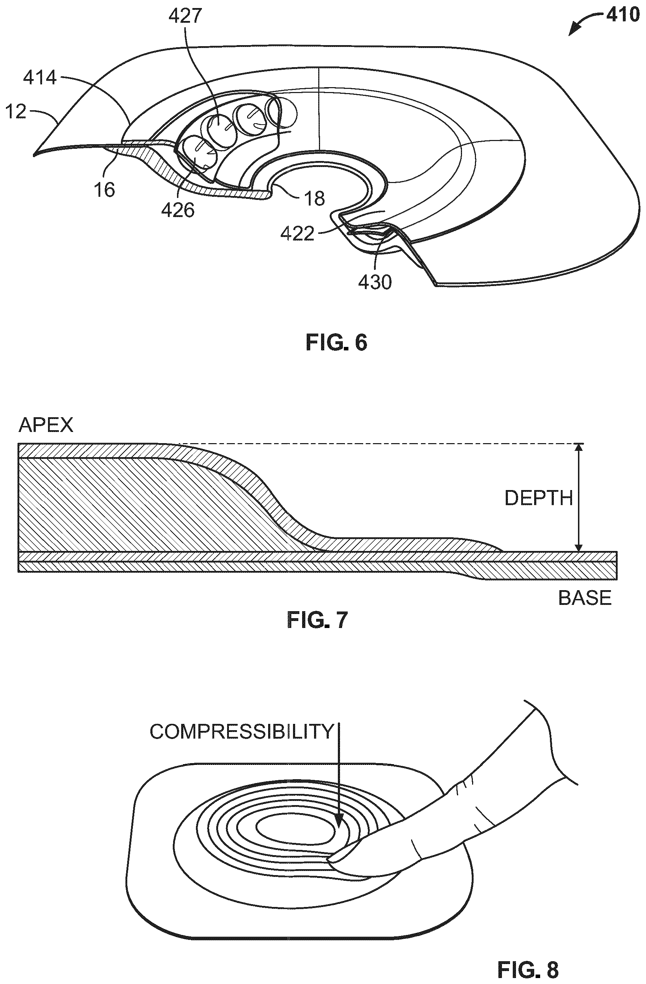

The characteristics of convexity of a skin barrier can include depth, compressibility, flexibility, tension location, and slope. *See,* McNichol, L., Cobb, T., Depaifve, Y., Quigley, M., Smitka, K., & Gray, M., Characteristics of Convex Skin Barriers and Clinical Application: Results of an International Consensus Panel, J Wound Ostomy Continence Nurs., (2021) 48(6), 524-532, Abstract. The depth of a convex skin barrier is defined as a distance from the apex of the dome to the base of the skin barrier. *Id,* at pg. 526. The depth D can be measured as a magnitude of the convexity from the base lying on the peristomal skin to the highest point of the skin barrier as shown in FIG. 7. Individual user's peristomal condition, such as depths of creases and folds around the stoma, should be carefully considered when determining a depth of a convex skin barrier to provide an optimal seal around the peristomal skin.

The compressibility of a convex skin barrier is defined as a capacity of the convex dome to be displaced or flattened as illustrated in FIG. 8. *Id*, at pg. 528. The compressibility may be measured as a force required to displace or flattened the dome portion of a convex skin barrier by a predetermined distance. A relatively easily compressible soft convex barrier may conform better to users with postoperative edema and/or a relatively firm abdomen. A relatively less compressible firm convex barrier may apply more pressure on the peristomal skin to provide support needed for users with a relatively soft abdominal tone and/or creases around the stoma. *Id.*

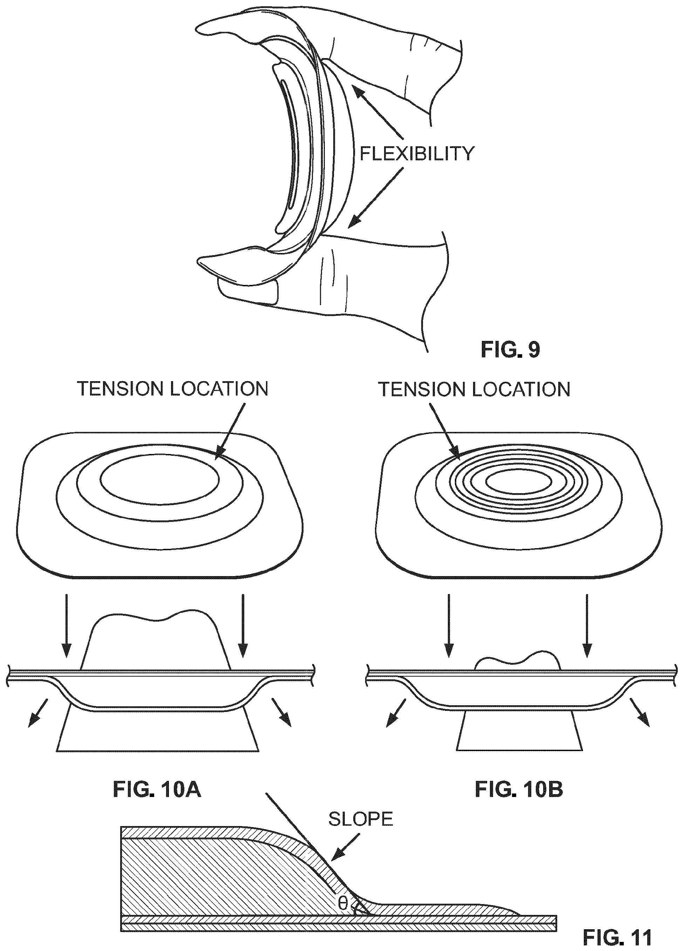

The flexibility of a convex skin barrier is defined as how easily the convex skin barrier can bend as illustrated in FIG. 9. at pg. 529. The flexibility is an important characteristic to consider when a skin barrier needs to bend to conform to abdominal contours. A relatively more flexible convex skin barrier may work well for users with multiple creases around stoma due to loose skin.

The tension location of a convex skin barrier is defined as the position in which a convex dome exerts downward and outward forces on the peristomal topography as illustrated in FIGS. 10A and 10B. *Id,* at pg. 530. A convex skin barrier configured to apply a tension close to a stoma may provide a consistent and reliable seal around the stoma that is flush to the skin or retraced below the skin. *Id.* For users with creases and folds around the stoma, a convex barrier skin barrier configured to apply a tension away from the stoma may help flatten the peristomal skin to provide a good seal.

The slope of a convex skin barrier is defined as an angle from the base of the dome to a periphery of the apex of the dome, as illustrated in FIG. 11. at pg. 53. Creases and folds around the stoma can compromise a seal between a skin barrier and the skin. Adjusting the slope of a convex skin barrier according to user's peristomal topography can improve the seal. For example, a convex skin barrier with a relatively small slope and wider plateau may help flatten the peristomal skin creases and folds to achieve a good seal.

Customizing and adjusting the depth, compressibility, flexibility, tension location, and/or slope of a convex skin barrier according to user's peristomal topography can provide an optimal seal around the stoma. The present disclosure provides an ostomy barrier appliance, including a convexity adjusting device configured to adjust one or more of the convexity characteristics of a skin barrier according to various embodiments.

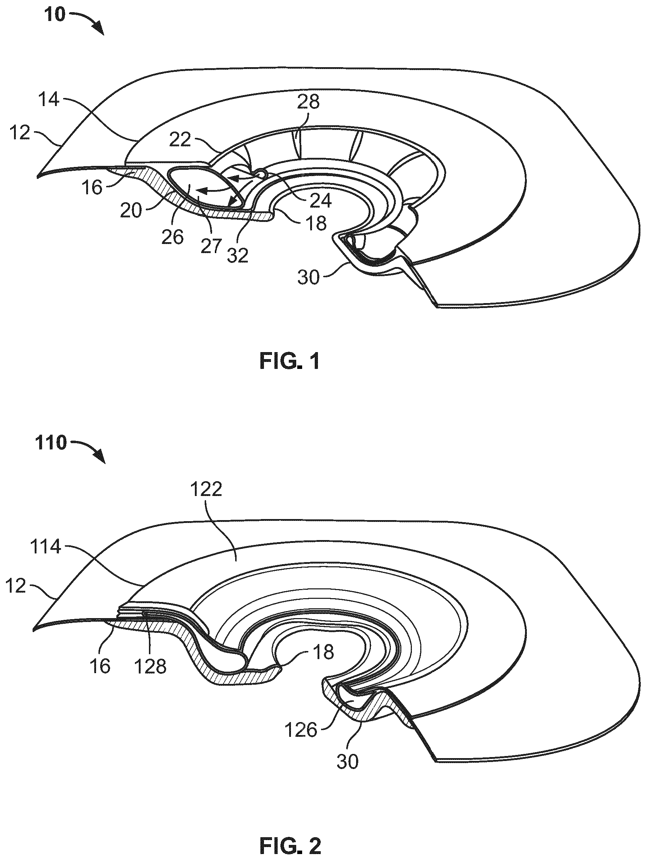

Referring now to the figures, FIGS. 1-6 show an ostomy barrier appliance according to several embodiments. FIG. 1 is a perspective view of the ostomy barrier appliance 10 according to an embodiment with a portion removed and viewed from a pouch-facing side (also referred to herein as a distal side) to illustrate a layered construction of the ostomy barrier appliance 10. The ostomy barrier appliance 10 may generally include a tape 12, a skin barrier 16, a convexity adjusting device 14, and an inlet opening 18 for receiving a stoma (not shown). The convexity adjusting device 14 may include an inflatable adjusting member 26 configured to adjust at least one characteristic of the convexity of the ostomy barrier appliance 10.

The tape 12 may include a first adhesive layer and a first backing layer. The first adhesive layer of the tape 12 may be formed from a suitable medical adhesive, such as an acrylic adhesive. The first backing layer may be formed from a suitable material, such as a nonwoven material or a thin polymeric film. In another embodiment, the tape 12 may comprise a hydrocolloid adhesive and a film backing layer.

In some embodiments, the skin barrier 16 may include a second backing layer laminated on the pouch-side surface of the skin barrier 16. The second backing layer may be formed from a suitable heat sealable polymeric material, such that the backing layer may be heat sealed to the tape 12. The skin barrier 16 may be formed from a suitable skin-contact adhesive, such as hydrocolloid adhesives.

In an embodiment, the convexity adjusting device 14 may be formed as a convex insert 14 comprising a base 22, an upper layer 20, and an inflatable adjusting member 26. The convex insert 14 may be configured to define and support a convexity of the skin barrier 16. The upper layer 20 may extend from the base 22 to an inner periphery 32, and the inflatable bladder 26 may be arranged adjacent a distal surface (pouch side surface) of the upper layer 20 between the base 22 and the upper layer 20.

In the embodiment of FIG. 1, the inflatable bladder 26 may be compartmentalized into a plurality of bladders 27, wherein each of the plurality bladders may be separately inflated or deflated to provide localized adjustment of a compressibility and/or depth of the convexity of the ostomy barrier appliance 10. Each of the plurality of bladders 27 may be separated from adjacent bladders by a sealed section 28 and may be inflated through a vessel 24.

In an embodiment, the ostomy barrier appliance 10 may be configured as a convex ostomy barrier including a dome 30 supported by the convex insert 14 including the inflatable bladder 26, where each of the plurality of bladders 27 may be inflated to provide a generally uniform initial compressibility and depth around the dome 30. In use, a user may deflate one or more of the plurality of bladders 27 according to user's peristomal topography to decrease the compressibility and/or depth in desired areas to create a seal that best fits user's need. Alternatively, the ostomy barrier appliance 10 may be provided each of the plurality of bladders 27 deflated or mostly deflated, and a user may inflate one or more of the plurality of bladders 27 to increase the compressibility and/or depth in desired areas. A user may inflate or deflate one or more of the plurality of bladder 27 by inserting or releasing a fluid, such as air, in a controlled amount to achieve the desired compressibility and/or depth.

FIGS. 2-6 show an ostomy barrier appliance 110, 210, 310, 410 according to several embodiments. The ostomy barrier appliance 110, 210, 310, 410, may be configured similar to ostomy barrier appliance 10 of FIG. 1, and may generally include a tape 12, a skin barrier 16, a convexity adjusting device 114, 314, 414, 414, and an inlet opening 18 for receiving a stoma. The convexity adjusting device 114, 214, 314 may include an inflatable bladder 126, 226, 326 which may be configured to adjust at least one characteristic of the convexity of the ostomy barrier appliance 110, 210, 310.

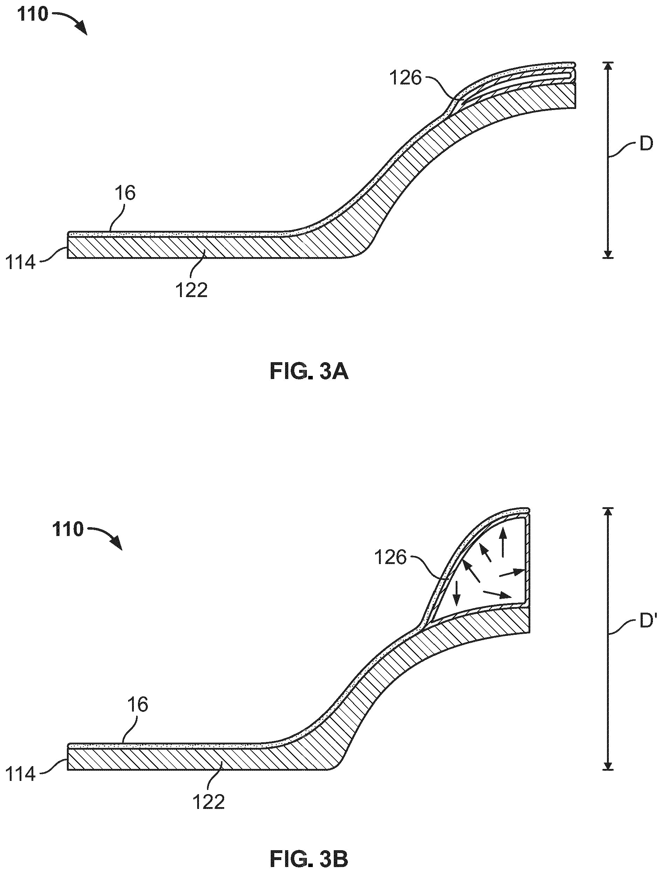

FIG. 2 is a perspective view of an ostomy barrier appliance 110 according to an embodiment. The ostomy barrier appliance 110 may include a convexity adjusting device 114 comprising a convex insert 122 and an inflatable bladder 126. In this embodiment, the inflatable bladder 126 may be arranged between the convex insert 122 and the skin barrier 16 in a dome area 30 and configured to adjust a convexity depth of the skin barrier 16. In an embodiment, the ostomy barrier appliance 110 may comprise a fluid path 128 configured to allow a fluid, such as air, to enter and exit the inflatable bladder 26. FIG. 3A illustrates the inflatable bladder 126 substantially deflated, wherein the depth D of the convexity of the ostomy barrier appliance 110 is substantially defined by the depth of the convex insert 122. FIG. 3B illustrates the inflatable bladder 126 inflated to increase the depth D' of the convexity, wherein the depth D' at an inflated state is greater than the depth D at a deflated state. The inflatable bladder 26 may be configured to be inflated or deflated by inserting or releasing an amount of fluid to provide a desired depth of the convexity of the ostomy barrier appliance 110.

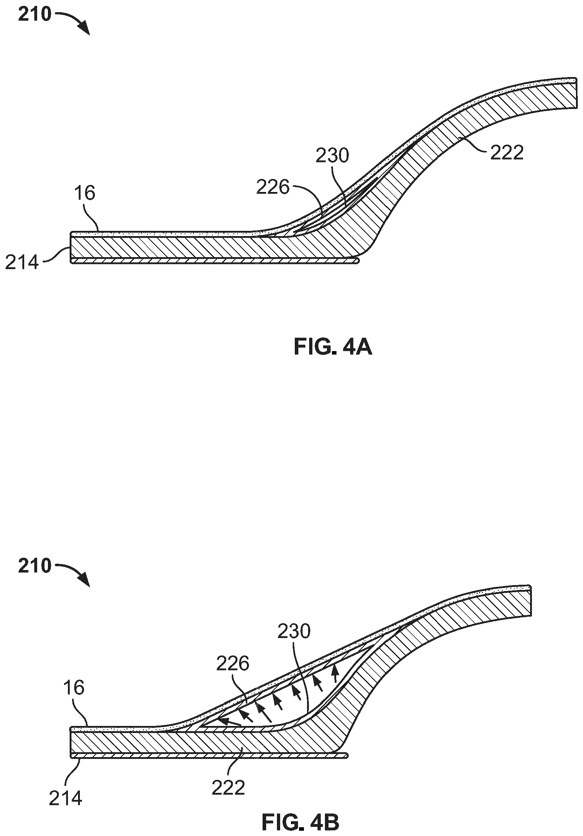

FIGS. 4A and 4B are cross-sectional views of an ostomy barrier appliance 210 according to another embodiment. Similar to the convex adjusting device 114, a convexity adjusting device 214 may comprise a convex insert 222 and an inflatable bladder 226. In this embodiment, the inflatable bladder 226 may be arranged between the convex insert 222 and the skin barrier 16 in a concave area 230 of the convex insert 222 and configured to adjust a convexity slope of the skin barrier 16. In FIG. 4A, the inflatable bladder 226 may be substantially deflated, wherein the convexity of the ostomy barrier appliance 210 including the slope is substantially defined by the convexity of the convex insert 222. In FIG. 4B, the inflatable bladder 226 is inflated to decrease the slope. As shown, the slope of the convex barrier appliance 210 is smaller or shallower when the inflatable bladder 226 is inflated (FIG. 4B) than when the inflatable bladder 226 is deflated (FIG. 4A). The inflatable bladder 226 may be configured to be inflated or deflated by inserting or releasing an amount of fluid, such as air, to provide a desired slope of the convexity of the ostomy barrier appliance 210.

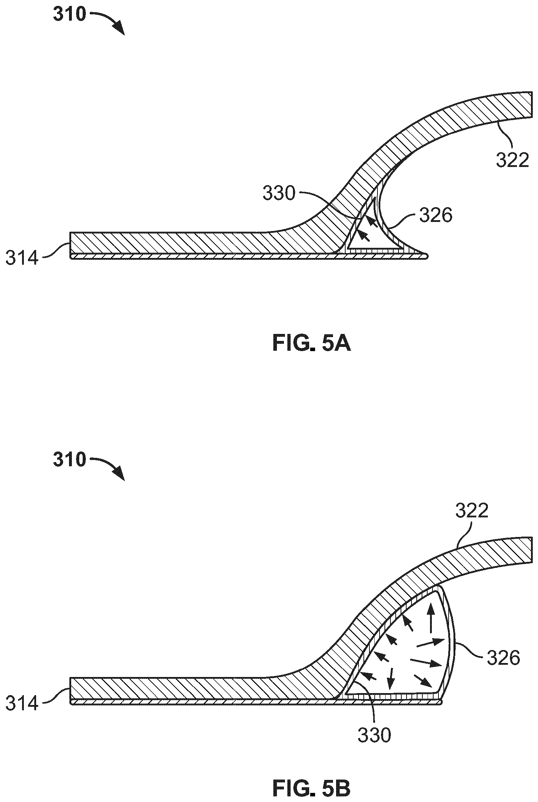

FIGS. 5A and 5B are cross-sectional views of an ostomy barrier appliance 310 according to an embodiment. Similar to the convex adjusting device 114, 214, a convexity adjusting device 314 may comprise a convex insert 322 and an inflatable bladder 326. In this embodiment, the inflatable bladder 326 may be arranged adjacent a distal surface (pouch side surface) of the convex insert 322 proximate a base area 330 and configured to adjust a compressibility of the convexity of the skin barrier 16. In an embodiment, the ostomy barrier appliance 310 may be configured such that the compressibility may be adjusted by inflating or deflating the inflatable bladder 326 while the depth and slope of the convexity defined by the convex insert 322 may remain unchanged. In another embodiment, the ostomy barrier appliance 310 may be configured such that the compressiblity, depth and/or slope of the convexity defined by the convex insert 322 may be ajusted by inflating or deflating the inflatable bladder 326. In FIG. 5A, the inflatable bladder 326 may be substantially deflated such that the compressibility of ostomy barrier appliance 310 may be substantially defined by the compressibility of the convex insert 322. In FIG, 5B, the inflatable bladder 326 may be inflated to increase the compressibility of the ostomy barrier appliance 310. The compressibility of the ostomy barrier appliance 310 when the inflatable bladder 326 is inflated may be greater or firmer when compared to when the inflatable bladder 326 is deflated. The inflatable bladder 326 may be configured to be inflated or deflated by inserting or releasing an amount of fluid, such as air, to provide a desired compressibility of the convexity of the ostomy barrier appliance 310.

FIG. 6 is a perspective view of an ostomy barrier appliance 410 according to an embodiment with a portion removed and viewed from a distal side. The ostomy barrier appliance 410 may be configured similar to the ostomy barrier appliance 210 comprising a convexity adjusting device 414 that includes a convex insert 422 and an inflatable bladder 426. The inflatable bladder 426 may be arranged between the convex insert 422 and the skin barrier 16 generally in a middle portion or in a concave portion 430 of the convex insert 422 and configured to adjust a convexity slope of the skin barrier 16. In this embodiment, the inflatable bladder 426 may include a plurality of bladders 427, each of which may be separately inflated or deflated to provide a localized adjustment of the convexity slope. In use, a user may inflate one or more of the plurality of bladders 427 to decrease the convexity slope or deflate one or more of the plurality of bladders 427 to increase the convexity slope at desired locations. In an embodiment, the inflatable bladder 426 may comprise a plurality of pockets 427 filled with air. In use, a user may compress or pop one or more of the plurality of air pockets 427 to increase the convexity slope at desired locations around the ostomy barrier appliance 410.

From the foregoing it will be observed that numerous modifications and variations can be effectuated without departing from the scope of the novel concepts of the present disclosure. It is to be understood that no limitation with respect to the specific embodiments illustrated is intended or should be inferred. The disclosure is intended to cover by the appended claims all such modifications as fall within the scope of the claims.

## Claims

1. A convex ostomy barrier appliance (10, 110, 210, 310, 410, 510) comprising:
a skin barrier (16);
an inlet opening for receiving the stoma; and
a convexity adjusting device (14) comprising an inflatable bladder (26) configured to adjust at least one characteristic of a convexity of the ostomy barrier appliance, **characterised in that** the inflatable bladder is compartmentalized into a plurality of bladders (27), wherein the convexity adjusting device is configured such that each of the plurality of bladders can be inflated or deflate separately.

2. The convex ostomy barrier appliance (10, 110, 210, 310, 410, 510) of claim 1, wherein each of the plurality of bladders (27) is separated from adjacent bladders by a sealed section (28).

3. The convex ostomy barrier appliance (10, 110, 210, 310, 410, 510) of claim 1 or 2, wherein the ostomy barrier appliance is provided with each of the plurality of bladders (27) inflated to provide a first convexity characteristic, wherein one or more of the plurality of bladders (27) is deflated to adjust the first convexity characteristic to a second convexity characteristic at one or more of corresponding locations to provide a localized and customized adjustment of the convexity characteristic.

4. The convex ostomy barrier appliance (10, 110, 210, 310, 410, 510) of claim 1 or 2, wherein the ostomy barrier appliance is provided with each of the plurality of bladders (27) deflated to provide a third convexity characteristic, wherein one or more of the plurality of bladders (27) is inflated to adjust the third convexity characteristic to a fourth convexity characteristic at one or more of corresponding locations to provide a localized and customized adjustment of the convexity characteristic.

5. The convex ostomy barrier appliance (10, 110, 210, 310, 410, 510) of any one of claims 1-4, further including a convex insert comprising a base (22) and an upper layer (20), wherein the upper layer extends from the base to an inner periphery (32) and the inflatable bladder is arranged adjacent a distal surface of the upper layer between the base (22) and the upper layer (20), wherein the inflatable bladder (26) is configured to provide a localized adjustment of a compressibility and/or a depth of the convexity by inflating or deflating one or more of the plurality of bladders (27).

6. The convex ostomy barrier appliance (10, 110, 210, 310, 410, 510) of any one of claims 1-4, further including a convex insert (122), wherein the inflatable bladder is arranged between the convex insert (122) and the skin barrier, wherein each of the plurality of bladders (27) is configured to be inflated or deflated separately to provide a localized and customized adjustment of a slope of the convexity.

7. The convex ostomy barrier appliance (10, 110, 210, 310, 410, 510) of claim 6, wherein the inflatable bladder (26) is arranged in a concave portion of the convex insert (122).

8. The convex ostomy barrier appliance (10, 110, 210, 310, 410, 510) of claim 6 or 7, wherein one or more of the plurality of bladders (27) is inflated to decrease the slope of the convexity at one or more corresponding locations.

9. The convex ostomy barrier appliance (10, 110, 210, 310, 410, 510) of claim 6 or 7, wherein one or more of the plurality of bladders (27) is deflated to increase the slope of the convexity at one or more corresponding locations.

10. The convex ostomy barrier appliance (10, 110, 210, 310, 410, 510) of claim 6 or 7, wherein the ostomy barrier appliance is provided with each of the plurality of bladders (27) inflated to provide a generally uniform slope around the convexity, wherein one or more of the plurality of bladders (27) can be compressed or popped to increase the slope of the convexity at one or more corresponding locations.

11. The convex ostomy barrier appliance (10, 110, 210, 310, 410, 510) of any one of claims 1-4, further including a convex insert (122), wherein the inflatable bladder is arranged between the convex insert and the skin barrier in a dome area (30) and configured to adjust a depth of the convexity.

12. The convex ostomy barrier appliance (10, 110, 210, 310, 410, 510) of claim 11, wherein the ostomy barrier appliance is provided with the inflatable bladder (26) substantially deflated to provide a first depth, wherein the inflatable bladder (26) is configured to be inflated by a user to increase the depth to a second depth, wherein the second depth is greater than the first depth.

13. The convex ostomy barrier appliance (10, 110, 210, 310, 410, 510) of claim 11, wherein the ostomy barrier appliance is provided with the inflatable bladder (26) inflated to provide a third depth, wherein the inflatable bladder (26) is configured to be deflated by a user to decrease the depth to a fourth depth, wherein the fourth depth is less than the third depth.

14. The convex ostomy barrier appliance (10, 110, 210, 310, 410, 510) of any of claims 1-13, wherein the inflatable bladder (26) is configured to be inflated by inserting a fluid into the inflatable bladder, and wherein the inflatable bladder (26) is configured to be deflated by releasing the fluid from the inflatable bladder (26).

15. The convex ostomy barrier appliance (10, 110, 210, 310, 410, 510) of any of claims 1-14, wherein the fluid is air.

## Patentansprüche

1. Konvexe Ostomiebarriereeinrichtung (10, 110, 210, 310, 410, 510), Folgendes umfassend:
eine Hautbarriere (16);
eine Einlassöffnung zum Aufnehmen der Ostomie; und
eine Vorrichtung (14) zum Anpassen der Konvexität, umfassend eine aufblasbare Blase (26), die dazu konfiguriert ist, mindestens ein Konvexitätsmerkmal der Ostomiebarriereeinrichtung anzupassen, **dadurch gekennzeichnet, dass** die aufblasbare Blase in eine Vielzahl von Blasen (27) unterteilt ist, wobei die Vorrichtung zum Anpassen der Konvexität so konfiguriert ist, dass jede der Vielzahl von Blasen separat aufgeblasen oder entleert werden kann.

2. Konvexe Ostomiebarriereeinrichtung (10, 110, 210, 310, 410, 510) nach Anspruch 1, wobei jede der Vielzahl von Blasen (27) durch einen abgedichteten Bereich (28) von benachbarten Blasen getrennt ist.

3. Konvexe Ostomiebarriere (10, 110, 210, 310, 410, 510) nach Anspruch 1 oder 2, wobei die Ostomiebarriereeinrichtung mit jeweils einer der Vielzahl von Blasen (27) bereitgestellt ist, die aufgeblasen ist, um ein erstes Konvexitätsmerkmal bereitzustellen, wobei eine oder mehrere der Vielzahl von Blasen (27) entleert sind, um das erste Konvexitätsmerkmal an einer oder mehreren entsprechenden Stellen an ein zweites Konvexitätsmerkmal anzupassen und so eine lokalisierte und individuelle Anpassung des Konvexitätsmerkmals bereitzustellen.

4. Konvexe Ostomiebarriereeinrichtung (10, 110, 210, 310, 410, 510) nach Anspruch 1 oder 2, wobei die Ostomiebarriereeinrichtung mit jeder der Vielzahl von Blasen (27) bereitgestellt ist, die entleert ist, um ein drittes Konvexitätsmerkmal bereitzustellen, wobei eine oder mehrere der Vielzahl von Blasen (27) aufgeblasen sind, um das dritte Konvexitätsmerkmal an einer oder mehreren entsprechenden Stellen an ein viertes Konvexitätsmerkmal anzupassen und so eine lokalisierte und individuelle Anpassung des Konvexitätsmerkmals bereitzustellen.

5. Konvexe Ostomiebarriereeinrichtung (10, 110, 210, 310, 410, 510) nach einem der Ansprüche 1-4, ferner einen konvexen Einsatz beinhaltend, der eine Basis (22) und eine obere Schicht (20) umfasst, wobei sich die obere Schicht von der Basis bis zu einem inneren Umfang (32) erstreckt und die aufblasbare Blase benachbart zu einer distalen Oberfläche der oberen Schicht zwischen der Basis (22) und der oberen Schicht (20) angeordnet ist, wobei die aufblasbare Blase (26) so konfiguriert ist, dass sie eine lokale Anpassung einer Kompressibilität und/oder einer Tiefe der Konvexität durch Aufblasen oder Entleeren einer oder mehrerer der Vielzahl von Blasen (27) bereitstellt.

6. Konvexe Ostomiebarriereeinrichtung (10, 110, 210, 310, 410, 510) nach einem der Ansprüche 1-4, ferner einen konvexen Einsatz (122) beinhaltend, wobei die aufblasbare Blase zwischen dem konvexen Einsatz (122) und der Hautbarriere angeordnet ist, wobei jede der Vielzahl von Blasen (27) so konfiguriert ist, dass sie separat aufgeblasen oder entleert werden kann, um eine lokalisierte und individuelle Anpassung der Neigung der Konvexität bereitzustellen.

7. Konvexe Ostomiebarriereeinrichtung (10, 110, 210, 310, 410, 510) nach Anspruch 6, wobei die aufblasbare Blase (26) in einem konkaven Abschnitt des konvexen Einsatzes (122) angeordnet ist.

8. Konvexe Ostomiebarriereeinrichtung (10, 110, 210, 310, 410, 510) nach Anspruch 6 oder 7, wobei eine oder mehrere der Vielzahl von Blasen (27) aufgeblasen sind, um die Neigung der Konvexität an einer oder mehreren entsprechenden Stellen zu verringern.

9. Konvexe Ostomiebarriereeinrichtung (10, 110, 210, 310, 410, 510) nach Anspruch 6 oder 7, wobei eine oder mehrere der Vielzahl von Blasen (27) entleert sind, um die Neigung der Konvexität an einer oder mehreren entsprechenden Stellen zu vergrößern.

10. Konvexe Ostomiebarriereeinrichtung (10, 110, 210, 310, 410, 510) nach Anspruch 6 oder 7, wobei die Ostomiebarriereeinrichtung mit jeder der Vielzahl von Blasen (27) bereitgestellt ist, die aufgeblasen ist, um eine im Allgemeinen gleichmäßige Neigung um die Konvexität herum bereitzustellen, wobei eine oder mehrere der Vielzahl von Blasen (27) komprimiert oder zum Platzen gebracht werden können, um die Neigung der Konvexität an einer oder mehreren entsprechenden Stellen zu erhöhen.

11. Konvexe Ostomiebarriereeinrichtung (10, 110, 210, 310, 410, 510) nach einem der Ansprüche 1-4, ferner einen konvexen Einsatz (122) beinhaltend, wobei die aufblasbare Blase zwischen dem konvexen Einsatz und der Hautbarriere in einem Kuppelbereich (30) angeordnet und dazu konfiguriert ist, die Tiefe der Konvexität anzupassen.

12. Konvexe Ostomiebarriereeinrichtung (10, 110, 210, 310, 410, 510) nach Anspruch 11, wobei die Ostomiebarriereeinrichtung mit der aufblasbaren Blase (26) bereitgestellt ist, die im Wesentlichen entleert ist, um eine erste Tiefe bereitzustellen, wobei die aufblasbare Blase (26) so konfiguriert ist, dass sie durch einen Benutzer aufgeblasen werden kann, um die Tiefe auf eine zweite Tiefe zu erhöhen, wobei die zweite Tiefe größer als die erste Tiefe ist.

13. Konvexe Ostomiebarriereeinrichtung (10, 110, 210, 310, 410, 510) nach Anspruch 11, wobei die Ostomiebarriereeinrichtung mit der aufblasbaren Blase (26) bereitgestellt ist, die aufgeblasen ist, um eine dritte Tiefe bereitzustellen, wobei die aufblasbare Blase (26) so konfiguriert ist, dass sie durch einen Benutzer entleert werden kann, um die Tiefe auf eine vierte Tiefe zu verringern, wobei die vierte Tiefe geringer als die dritte Tiefe ist.

14. Konvexe Ostomiebarriereeinrichtung (10, 110, 210, 310, 410, 510) nach einem der Ansprüche 1-13, wobei die aufblasbare Blase (26) so konfiguriert ist, dass sie durch Einleiten eines Fluids in die aufblasbare Blase aufgeblasen wird, und wobei die aufblasbare Blase (26) so konfiguriert ist, dass sie durch Ablassen des Fluids aus der aufblasbaren Blase (26) entleert wird.

15. Konvexe Ostomiebarriereeinrichtung (10, 110, 210, 310, 410, 510) nach einem der Ansprüche 1-14, wobei das Fluid Luft ist.

## Revendications

1. Appareil de barrière de stomie convexe (10, 110, 210, 310, 410, 510), comprenant :
une barrière cutanée (16) ;
une ouverture d'entrée destinée à recevoir la stomie ; et
un dispositif de réglage de convexité (14) comprenant une vessie gonflable (26) conçue pour régler au moins une caractéristique de la convexité de l'appareil de barrière de stomie, **caractérisé en ce que** la vessie gonflable est compartimentée en une pluralité de vessies (27), dans lequel le dispositif de réglage de convexité est conçu de sorte que chacune de la pluralité de vessies puisse être gonflée ou dégonflée séparément.

2. Appareil de barrière de stomie convexe (10, 110, 210, 310, 410, 510) selon la revendication 1, dans lequel chacune de la pluralité de vessies (27) est séparée des vessies adjacentes par une section scellée (28).

3. Appareil de barrière de stomie convexe (10, 110, 210, 310, 410, 510) selon la revendication 1 ou 2, dans lequel l'appareil barrière de stomie est muni de chacune de la pluralité de vessies (27) gonflées pour fournir une première caractéristique de convexité, dans lequel une ou plusieurs de la pluralité de vessies (27) sont dégonflées pour régler la première caractéristique de convexité sur une deuxième caractéristique de convexité à un ou plusieurs emplacements correspondants afin d'assurer un réglage localisé et personnalisé de la caractéristique de convexité.

4. Appareil de barrière de stomie convexe (10, 110, 210, 310, 410, 510) selon la revendication 1 ou 2, dans lequel l'appareil barrière de stomie est pourvu de chacune de la pluralité de vessies (27) dégonflées pour assurer une troisième caractéristique de convexité, dans lequel une ou plusieurs de la pluralité de vessies (27) sont gonflées pour régler la troisième caractéristique de convexité sur une quatrième caractéristique à un ou plusieurs emplacements correspondants afin d'assurer un réglage localisé et personnalisé de la caractéristique de convexité.

5. Appareil de barrière de stomie convexe (10, 110, 210, 310, 410, 510) selon l'une quelconque des revendications 1 à 4, comportant en outre un insert convexe comprenant une base (22) et une couche supérieure (20), dans lequel la couche supérieure s'étend de la base à une périphérie interne (32) et la vessie gonflable est agencée à côté d'une surface distale de la couche supérieure entre la base (22) et la couche supérieure (20), dans lequel la vessie gonflable (26) est conçue pour assurer un réglage localisé d'une compressibilité et/ou d'une profondeur de la convexité par gonflage ou dégonflage d'une ou de plusieurs de la pluralité de vessies (27).

6. Appareil de barrière de stomie convexe (10, 110, 210, 310, 410, 510) selon l'une quelconque des revendications 1 à 4, comportant également un insert convexe (122), dans lequel la vessie gonflable est agencée entre l'insert convexe (122) et la barrière cutanée, dans lequel chacune de la pluralité de vessies (27) est conçue pour être gonflée ou dégonflée séparément afin d'assurer un réglage localisé et personnalisé d'une pente de la convexité.

7. Appareil de barrière de stomie convexe (10, 110, 210, 310, 410, 510) selon la revendication 6, dans lequel la vessie gonflable (26) est agencée dans une partie concave de l'insert convexe (122).

8. Appareil de barrière de stomie convexe (10, 110, 210, 310, 410, 510) selon la revendication 6 ou 7, dans lequel une ou plusieurs de la pluralité de vessies (27) sont gonflées pour diminuer la pente de la convexité à un ou plusieurs emplacements correspondants.

9. Appareil de barrière de stomie convexe (10, 110, 210, 310, 410, 510) selon la revendication 6 ou 7, dans lequel une ou plusieurs de la pluralité de vessies (27) sont dégonflées pour augmenter la pente de la convexité à un ou plusieurs emplacements correspondants.

10. Appareil de barrière de stomie convexe (10, 110, 210, 310, 410, 510) selon la revendication 6 ou 7, dans lequel l'appareil de barrière de stomie est pourvu de chacune de la pluralité de vessies (27) gonflées pour fournir une pente globalement uniforme autour de la convexité, dans lequel une ou plusieurs de la pluralité de vessies (27) peuvent être comprimées ou éclatées pour augmenter la pente de la convexité à un ou plusieurs emplacements correspondants.

11. Appareil de barrière de stomie convexe (10, 110, 210, 310, 410, 510) selon l'une quelconque des revendications 1 à 4, comportant également un insert convexe (122), dans lequel la vessie gonflable est agencée entre l'insert convexe et la barrière cutanée dans une zone en dôme (30) et conçue pour régler une profondeur de la convexité.

12. Appareil de barrière de stomie convexe (10, 110, 210, 310, 410, 510) selon la revendication 11, dans lequel l'appareil de barrière stomie est pourvu de la vessie gonflable (26) sensiblement dégonflée pour fournir une première profondeur, dans lequel la vessie gonflable (26) est conçue pour être gonflée par un utilisateur afin d'augmenter la profondeur jusqu'à une deuxième profondeur, dans lequel la deuxième profondeur est supérieure à la première profondeur.

13. Appareil de barrière de stomie convexe (10, 110, 210, 310, 410, 510) selon la revendication 11, dans lequel l'appareil de barrière de stomie est pourvu de la vessie gonflable (26) gonflée pour fournir une troisième profondeur, dans lequel la vessie gonflable (26) est conçue pour être dégonflée par un utilisateur afin de diminuer la profondeur jusqu'à une quatrième profondeur, dans lequel la quatrième profondeur est inférieure à la troisième profondeur.

14. Appareil de barrière de stomie convexe (10, 110, 210, 310, 410, 510) selon l'une quelconque des revendications 1 à 13, dans lequel la vessie gonflable (26) est conçue pour être gonflée en insérant un fluide dans la vessie gonflable, et dans lequel la vessie gonflable (26) est conçue pour être dégonflée en libérant le fluide de la vessie gonflable (26).

15. Appareil de barrière de stomie convexe (10, 110, 210, 310, 410, 510) selon l'une quelconque des revendications 1 à 14, dans lequel le fluide est l'air.
